Europäisches Patentamt

European Patent Office

Office européen des brevets

(11) Veröffentlichungsnummer : **0 420 880 B1**

(12) **EUROPÄISCHE PATENTSCHRIFT**

(45) Veröffentlichungstag der Patentschrift :
26.05.93 Patentblatt 93/21

(51) Int. Cl.⁵ : **A61K 49/00, A61K 49/02**

(21) Anmeldenummer : **89906726.8**

(22) Anmeldetag : **13.06.89**

(86) Internationale Anmeldenummer :
**PCT/DE89/00393**

(87) Internationale Veröffentlichungsnummer :
**WO 89/12465 28.12.89 Gazette 89/30**

(54) **VERFAHREN ZUR BILDLICHEN DARSTELLUNG VON TUMOREN UNTER VERWENDUNG VON MONOKLONALEN ANTIKÖRPERN.**

(30) Priorität : **13.06.88 DE 3820452**

(43) Veröffentlichungstag der Anmeldung :
**10.04.91 Patentblatt 91/15**

(45) Bekanntmachung des Hinweises auf die
Patenterteilung :
**26.05.93 Patentblatt 93/21**

(84) Benannte Vertragsstaaten :
**AT BE CH DE FR GB IT LI LU NL SE**

(56) Entgegenhaltungen :
**US-A- 3 927 193**
**Patnet Abstracts of Japan, Band 12, nr. 155 (C-494) (3002), 12 Mai 1988 & JP, A 62267241 (WAKUNAGA PHARMACEUT. CO. LTD) 19 November 1987, siehe Zusammenfassung**

(56) Entgegenhaltungen :
**Cancer, Band 54, nr. 7, 1 Oktober 1984, B.D. Mann et al.: "Imaging of human tumor xenografts in nude mice with radiolabeled monoclonal antibodies", Seiten 1318-1327, siehe den ganzen Artikel**
**Cancer Research, Band 40, August 1980, F.H. DeLand et al.: "Lymphoscintigraphy withradionuclide-labeled antibodies to carcinoembryonic antigen", siehe Seiten 2997-3000**
**The New England Journal of Medicine, Band 298, Nr. 25, 22 Juni 1978, D.M. Goldenberg et al.: "Use of radio-labeled antibodies to carcinoembryonic antigen for the detection and localization of diverse cancers by external photoscanning", siehe Seiten 1384-1388**

(73) Patentinhaber : **SCHERING AKTIENGESELLSCHAFT**
**Müllerstrasse 170/178**
**W-1000 Berlin 65 (DE)**

(72) Erfinder : **SPECK, Ulrich**
**Benediktinerstrasse 50**
**W-1000 Berlin 28 (DE)**
Erfinder : **CONRAD, Jürgen**
**Sonnenallee 70**
**W-1000 Berlin 44 (DE)**

EP 0 420 880 B1

**Beschreibung**

Die Erfindung betrifft ein Verfahren zur bildlichen Dar-Stellung von Tumoren unter Verwendung von monoklonalen Antikörpern.

Angesichts steigender Zahlen von Tumorerkrankungen besteht ein besonderer Bedarf an schonenden Diagnostikverfahren. Die bislang verwendeten Methoden weisen häufig den Mangel auf, daß die eingesetzten Kontrastmittel zur Darstellung von Tumoren einerseits zu spezifisch sind, d.h. nur mit einer Tumorart reagieren und häufig nur mit einem Teil der Tumorzellen, andererseits zu unspezifisch sind und mit anderen Geweben kreuzreagieren.

Einen wesentlichen Fortschritt in der Darstellung von Tumoren ergab sich aus der Entwicklung monoklonaler Antikörper und der Möglichkeit, diese in einheitlicher Spezifität und in großen Mengen herzustellen.

So wurde beispielsweise für die Kernspintomographie die Verwendung spezifischer "paramagnetischer" mAK als Kontrastmittel für den frühzeitigen Nachweis von Tumoren, das "staging" und die Therapiekontrolle vorgeschlagen, McNamara, M.T. et al., Radiology 153, 292 (1984); Unger,E.C. et al., Invest, Radiol. 20, 693 (1985).

In der EP 0074279 B 1 werden selektive Antitumormittel beschrieben, die einen Wirkstoff enthalten, der chemisch mit einem Antikörper verknüpft wurde. Dabei werden ebenfalls monoklonale Antikörper eingesetzt, die bezüglich des Tumorantigens spezifisch sind.

Bei den bisherigen Untersuchungen und Entwicklungen neuer Verfahren wurde jeweils eine möglichst hohe Spezifität der Antikörper für charakteristische Antigene angestrebt.

Dieses Vorgehen beinhaltet insofern auch Nachteile , da insbesondere auf Tumorzellen die Zahl der für die Bindung des Antikörpers entscheidenen Antigene beschränkt ist und außerdem die Antigene bei Fortschreiten der Erkrankung modulieren können. Die Lösung dieses Problems wurde bislang in der Auswahl von möglichst zuverlässig und in hoher Zahl auf den Tumorzellen auftretenden Antigenen gesehen. Diese Antigene sollten an keiner anderen Körperstelle in ähnlicher Konzentration auftreten. Ferner sollten die Antikörper selbst eine möglichst hohe Spezifität und Affinität gegenüber dem oder den ausgewählten Antigenen besitzen. Reichte die Konzentration eines Antigens auf der Oberfläche der betreffenden Tumorzellen nicht aus, um hinreichende Mengen der Antikörper zu binden, oder trat ein Antigen nicht zuverlässig genauf auf den Tumorzellen auf, so wurde versucht, dieses Problem durch die Anwendung eines Gemisches aus mehreren Antikörpern zu lösen.

Die zuvor genannten Methoden zur Darstellung von Tumoren besitzen alle den Nachteil, duß mit spezifischen monoklonalen Antikörpern eine rasche Absättigung der tumorassoziierten Antigene eintreten kann. Sofern also die Zahl der entsprechenden Antigene auf dem Tumor nicht hinreichend groß ist, wird die Konzentration der mAK eventuell zu gering für eine bildliche Darstellung der Tumore. Ein weiterer Nachteil der beschriebenen Verfahren liegt darin, daß die mAK unter dem Gesichtspunkt hoher Spezifität ausgewählt sein müssen. Dies hat zur Folge, daß Tumore nur dann und solange dargestellt werden können, soweit sie die zum verwendeten mAK korrespondierenden Antigene tragen. Andersartige Tumore, die diese Antigene nicht exprimieren, werden übersehen.

Aufgabe der Erfindung ist somit die Entwicklung eines verbesserten universellen Verfahrens zur bildlichen Darstellung von Tumoren möglichst unabhängig von den immunologischen Eigenschaften der Tumore.

Erfindungsgemäß wird diese Aufgabe dadurch gelöst, daß die verwendeten monoklonalen Antikörper keine definierte Bindungsspezifität hinsichtlich der tumorassoziierten Antigene aufweisen.

Gegenstand der Erfindung ist somit ein Verfahren zur bildlichen Darstellung von Tumoren unter Verwendung von Konjugaten monoklonaler Antikörper oder deren Fragmenten mit Metallkomplexen oder mit jodierten Verbindungen, das sich dadurch auszeichnet, daß die verwendeten monoklonalen Antikörper oder deren Fragmente keine definierte Bindungsspezifität hinsichtlich der tumorassoziierten Antigene aufweisen und die in den Komplexen vorhandenen Metalle Chrom (III), Mangan (II), Eisen (II), Cobalt (II), Nickel (II), Kupfer (II), Praseodym (III), Neodym (III), Samarium (III), Ytterbium (III), Gadolinium (III), Terbium (III), Dysprosium (III), Holmium (III), Erbium (III) oder Eisen (III) sind.

Es wurde beobachtet, daß die für den betreffenden Tumor nicht spezifischen Antikörper doch in gewissem Umfang in das Tumorgewebe aufgenommen werden, Mann, B.D. et al., Cancer 54, 1318 (1984).
Dieser Befund ist an sich nicht überraschend, da als Referenzgewebe für die Aufnahme von Antikörpern jeweils das Muskelgewebe gewählt wurde. Gegenüber dem Muskel erreichen sehr viele, auch niedermolekulare Substanzen, in sehr vielen besser vaskularisierten und mit einem größeren Extrazellularraum ausgestatteten Geweben eindeutig höhere Konzentrationen.

In der Nukleardiagnostik gelang eine gute bildliche Darstellung der Tumoren mit unspezifischen Antikörpern nur bei exzessiv großen und außerhalb des Abdomens lokalisierten Tumoren. Die Dosis der Antikörper und ihre Konzentration in den Tumoren waren sehr klein. Dies ist für die Nukleardiagnostik hinreichend, liegt jedoch weit unter den Anforderungen an Dosis bzw. Konzentration für andere bildgebende Verfahren.

Die Problematik bei der Nutzung von monoklonalen Antikörpern als Kontrastmittel für die Kernspintomographie liegt insofern anders, als sehr viel höhere Konzentrationen der Antikörper bzw. der für die Beeinflussung der Relaxationszeiten notwendigen paramagnetischen Elemente benötigt werden, als in der Nuklearmedizin. Bei der Untersuchung der Dosisabhängigkeit der Anreicherung von Antikörpern in Tumoren stellte sich überraschend heraus, daß es

a) zu keiner Absättigung der Aufnahme "unspezifischer" Antikörper in dem für spezifische Antikörper bekannten niedrigen Dosis/Konzentrationsbereich kam,

b) andererseits die Aufnahme der Antikörper in die Tumoren auch nicht völlig unspezifisch ist, da weder mit niedermolekularen (z.B. GdDTPA) noch mit hochmolekularen (GdDTPA-Albumin, Gd-gebunden an polymere Komplexbildner) Verbindungen gleiche Anreicherungen erzielt wurden,

c) im Gegensatz zu dem Beobachtungen von Mann et al., Cancer 54, 1318 (1984), die Konzentration der paramagnetisch markierten Antikörper auch in sehr kleinen, im Thorax oder Abdomen gelegenen Tumoren hoch genug war, um sie von inneren Organen (Darm, Lunge etc.) abzugrenzen.

Wenn auch die Konzentrationsunterschiede zwischen dem Tumor und dem normalen Gewebe für den unspezifiechen Antikörper in den meisten Fällen nicht ganz so hoch sind wie für spezifische Antikörper, so bieten die unspezifischen Antikörper doch eine Reihe von Vorteilen, die ihre Anwendung in vielen diagnostischen und therapeutischen Fällen gegenüber der Anwendung von spezifischen Antikörpern als vorteilhaft erscheinen läßt:

1. Die Anreicherung ist nicht abhängig von der Bindung an die nur in begrenzter Zahl vorhandenen tumorassöziierten Antigene. Daher erfolgt keine so rasche Absättigung wie im Falle der spezifischen mAK. Somit sind die beispielsweise für die Kernspintomographie notwendigen höheren Konzentratonen eher erreichbar.

2. Aufgrund der geringen oder fehlenden Spezifität der verwendeten Antikörper erfolgt eine Anreicherung weitgehend unabhängig von der Art des Tumors. Geringe Veränderungen der Antigenität von Primärtumor oder Metastasen führen nicht zu einem Verlust an Wirksamkeit des betreffenden Tracers.

3. Da zahlreiche unspezifische Antikörper zur Verfügung stehen, kann die Auswahl unter anderen Gesichtspunkten wie Herstellung, Reinheit, Stabilität und Verträglichkeit ,insbesondere der Immunogenität, umso sorgfältiger erfolgen.

4. Die Anreicherung von tumorunspezifischen mAK erlaubt auch den Einsatz von Zweikomponenten-Diagnostika. Beispielsweise können Antikörper verwendet werden, , die eine Spezifität für Metallkomplexe besitzen. Wenn diese Antikörper relativ hoch dosiert intravenös appliziert werden und anschließend nach Absinken des Blutspiegels Metallkomplexe appliziert werden, die über die Nieren filtrierbar sind, so erfolgt durch die Bindung an die Metallkomplex-spezifischen Antikörper eine Anreicherung der Metallkomplexe im Tumor.

5. Eine weitere Möglichkeit der Zweikomponenten-Diagnostika besteht darin, daß man im ersten Schritt mit Biotin oder Avidin konjugierte mAK einsetzt. Anschließend werden dann an Avidin gekoppeltes GdDTPA oder Biotin-Metall-Polymere appliziert.

Bei den vorliegenden Untersuchungen hat sich gezeigt, daß sich durchaus unterschiedliche Antikörper zur Diagnostik anwenden lassen. Dies ist unabhängig von der Bindungsspezifität der antigenbindenden Stellen. Dabei werden unterschiedliche Konzentrationen der Antikörper auf den Tumoren erreicht, und zwar in Abhängigkeit von der Tumorart und dem Tumorstadium.

Im erfindungsgemäßen Verfahren werden vorzugsweise monoklonale Antikörper murinen oder humanen Ursprungs verwendet. Es können auch lediglich Fragmente, beispielsweise in Form von Metallkomplexen, eingesetzt werden. Ferner können die mAK oder deren Fragmente jodiert sein. In einer weiteren bevorzugten Ausführungsform des erfindungsgemäßen Verfahrens sind die monoklonalen Antikörper oder deren Fragente über die Reste -NH$_2$, -SH, -COOH öder -CHO an Avidin gekoppelt und werden in Form der entsprechenden Konjugate eingesetzt.

Das erfindungsgemäße Verfahren kann in einer weiteren Ausführungsform in zwei Schritten durchgeführt werden. Dabei werden im ersten Schritt monoklonale Antikörper eingesetzt, die eine Spezifität für ein diagnostisches Mittel, z.B. einen Metallkonplex, aufweisen. Diese Antikörper werden relativ hoch dosiert i.v. appliziert. Nachdem der Blutspiegel abgesunken ist, können dann die Metallkomplexe, z.B. Gd-Komplexe, die über die Nieren filtrierbar sind, appliziert werden. Sie reichern sich im Tumor an, da sie von den Gd-Komplex-spezifischen Antikörpern zurückgehalten werden. Da sich das erfindungsgemäße Verfahren besonders in Zusammenhang mit der NMR-Diagnostik eignet, müssen die Zentralionen paramagnetisch sein.

Geeignet sind insbesondere Metallkomplexe von chrom (III), (Mangan (II), Eisen (II), Cobalt (II), Nickel (II), Kupfer (II), Praseodym (III), Neodym (III), Samarium (III), Ytterbium (III), Gadolinium (III), Terbium (III), Dysprosium (III), Holmium (III), Erbium (III) oder Eisen (III).

Man kann auch mittels Biotin oder Avidin modifizierte mAK einsetzen. In diesem Falle werden im zweiten Schritt dann an Avidin gekoppeltes GdDTPA oder Biotin-Gd- Polymere appliziert.

Das erfindungsgemäße Verfahren eignet sich besonders gut für die Kernspintomographie. Jedoch läßt es sich auch im Zusammenhang mit anderen Diagnostik-Verfahren anwenden, wie beispielsweise bei der Radiodiagnostik oder der Röntgendiagnostik.

Es ist klar, daß anstelle der Diagnostika nach dem erfindungsgemäßen Verfahren auch therapeutisch wirksame Mittel an ihren Zielort, dem Tumor, transportiert werden können. Dazu zählen auch Strahlung abgebende Substanzen, die das Tumorgewebe gezielt zerstören.

**BEISPIELE**

Beispiel 1:

Je 9 Nacktmäusen (Balb/c nu/nu) mit subkutan implantierten MX-1 Mammacarcinomen (Kontrollen: HT 29 Coloncarcinom) wurden jeweils 7 µg jodierte ($^{125}$J) mammacarcinom-spezifische Antikörper (788 G 6, 697 B 3), gelöst in 0,3 ml Natriumacetat - puffer (20 mM NaAc, 150 mM NaCl, p.H. 6,5) i.v. appliziert. Nach 48 Stunden wurden die Tiere getötet und folgende Organe entnommen: Blut, Leber, Nieren, Milz, Muskel, Knochen und Tumor.

Es wurden die Organkonzentrationen bestimmt und die Tumor-Gewebe-Quotienten berechnet.

| Tabelle 1 Tumor-Gewebe-Quotienten | $= \dfrac{\text{\% Dosis /g Tumor}}{\text{\% Dosis /g Gewebe}}$ | | | |
|---|---|---|---|---|
| | MX-1 Tumor | | HT 29 Tumor | |
| | mAK 788 G6 | mAK 697 B3 | mAK 788 G6 | mAK 697 B3 |
| Blut | 0.679 ± 0.185 | 0.713 ± 0.113 | 0.337 ± 0.040 | 0.353 ± 0.056 |
| Leber | 3.34 ± 1.45 | 3.79 ± 0.54 | 1.80 ± 1.10 | 1.70 ± 0.38 |
| Nieren | 4.13 ± 0.87 | 4.39 ± 0.53 | 2.10 ± 0.24 | 2.13 ± 0.47 |
| Milz | 4.13 ± 1.41 | 4.55 ± 0.74 | 1.99 ± 0.25 | 2.00 ± 0.33 |
| Muskel | 7.29 ± 2.68 | 9.21 ± 2.46 | 4.13 ± 0.96 | 4.81 ± 1.42 |
| Knochen | 7.48 ± 2.70 | 7.90 ± 1.63 | 3.07 ± 0.35 | 3.49 ± 0.72 |

Die Tabelle zeigt, daß es sowohl im antikörperspezifischen Tumor (MX-1), als auch im Kontrolltumor (HT 29) zu einer Anreicherung der Antikörper kommt.

Kontrollversuch zu Beispiel 1 mit RSA (DTPA $^{153}$ Gd)$_{36}$ 6 Nacktmäusen (Balb/c nu/nu) mit subkutan implantierten HT 29-Coloncarcinomen wurden jeweils 50 µg BSA (DTPA $^{153}$ Gd)$_{36}$ (bezogen auf die Proteinkonzentration) gelöst in 0,3 ml 0,9 % Kochsalzlösung i.v.appliziert. Nach 4 und 48 Stunden wurden jeweils 3 Tiere getötet. Es wurden Blut, Leber, Milz, Nieren, Knochen, Muskel und Tumor entnommen und die $^{153}$ Gd-Konzentration in den Geweben bestimmt. Dann wurden die Tumor-Gewebe Quotienten bestimmt.

BSA = Rinderserumalbumin

4

## Tabelle 2

Tumor-Gewebe-Quotienten = $\dfrac{\text{\% der Dosis/g Tumor}}{\text{\% der Dosis/g Gewebe}}$

| Gewebe | 4 h | 48 h |
|---|---|---|
| Blut | $2,16 \pm 1,08$ | $17,1 \pm 2,48$ |
| Leber | $0,03 \pm 0,01$ | $0,03 \pm 0,01$ |
| Milz | $0,09 \pm 0,02$ | $0,08 \pm 0,03$ |
| Muskel | $4,58 \pm 1,81$ | $3,23 \pm 2,25$ |
| Niere | $0,18 \pm 0,06$ | $0,34 \pm 0,05$ |
| Knochen | $0,12 \pm 0,02$ | $0,07 \pm 0,01$ |

Nur gegenüber Muskel und Blut zeigt sich eine Anreicherung im Tumor. Die Konzentrationen in den anderen untersuchten Organen liegen über der des Tumors.

Beispiel 2:

Je 3 Nacktmäusen mit MX-1 Mammacarcinom (Kontrollen: HT 29 Coloncarcinom) wurden jeweils 9, 5 μg jodierte coloncarcinomspezifische Antikörper (1083-17-1A) gelöst in 0,3 ml plysiologische Kochsalzlösung i.v. appliziert.
Nach 48 Std. wurden die Tiere getötet und folgende Organe entnommen: Blut, Leber, Nieren, Milz, Muskel, Knochen und Tumor.
Es wurden die Organkonzentrationen bestimmt und die Tumor-Gewebe-Quotienten berechnet.

EP 0 420 880 B1

Tabelle 3    % der Dosis pro g Tumor
            ─────────────────────── = Tumor-Gewebe-Quotienten
             % der Dosis pro g Gewebe


|        | MX - 1 Tumor | HT 29 Tumor |
|--------|--------------|-------------|
| Blut   | 0,67         | 0,51        |
| Leber  | 3,33         | 2,72        |
| Nieren | 3,04         | 2,18        |
| Milz   | 3,10         | 2,36        |
| Muskel | 8,03         | 6,67        |
| Knochen| 5,2          | 4,13        |

Die Tabelle zeigt, daß sich der coloncarcinomspezifische Antikörper in beiden Tumoren gleichermaßen anreichert.

Beispiel 3:

Es wurde die Anreicherung eines nicht tumorspezifischen mAK in Tumoren bei Nacktmäusen untersucht.
Antikörper:    DP 43-16 Product Code MCA 220 Camon Laborservice GmbH, Wiesbaden
Antigen:       Neurofilament ( 160 K ) vom Menschen
Tumore :       HPM 73 (Melanom) HT 29 Coloncarcinom
Es wurden 4 Dosierungen (5 μg, 50 μg, 250 μg, 1 mg pro Tier, 3 Tiere pro Dosis) i.v. appliziert.
Der Antikörper lag radio jodiert vor ( $^{125}$ J). Er wurde in 300 μl 0,9% NaCl-Lösung injiziert. In Stoffwechselkäfigen wurdem Harn und Faeces 4, 24 und 48 Stunden nach der Applikation gesammelt. Nach 48 Stunden wurden die Tiere getötet und folgende Organe entnommen: Blut, Leber, Nieren, Milz, Muskel, Tumor, Knochen und Knochenmark. Der Restkörper wurde in KOH gekocht. Die Messung der Aktivität erfolgte in einem Gamma Counter. Tabelle 4 a zeigt die Konzentrationen des Antikörpers in den einzelnen Organen. Die Gewebskonzentrationen steigen linear mit der Dosis an. Die Tumor-Gewebe-Quotienten (Tabelle 4 b) verändern sich nicht.
Es tritt in den für eine NMR-Diagnostik relevanten Antikörperkonzentrationen im Tumorgewebe keine Sättigung ein, so daß eine Differenzierung in Tumor und Nichttumorgewebe aufgrund der in allen Dosierungen höheren Tumorkonzentraton möglich ist.

6

## Tabelle 4 a

Konzentrationen des Antikörpers in den einzelnen Organen

(% Dosis./ g Organ)

|  | Dosis I (5 µg/Tier) | Dosis II (50 µg/Tier) | Dosis III (250 µg/Tier) | Dosis IV (1 mg/Tier) |
|---|---|---|---|---|
| Blut | 16,32 +/- 4,35 | 18,55 +/- 4,62 | 15,28 +/- 0,90 | 17,35 +/- 0,66 |
| Leber | 3,57 +/- 1,54 | 4,22 +/- 1,54 | 3,44 +/- 0,16 | 3,83 +/- 0,38 |
| Nieren | 3,10 +/- 1,01 | 3,84 +/- 0,89 | 3,42 +/- 0,06 | 3,63 +/- 0,37 |
| Milz | 2,71 +/- 1,24 | 2,82 +/- 0,80 | 2,49 +/- 0,20 | 3,13 +/- 0,02 |
| Muskel | 1,19 +/- 0,40 | 1,47 +/- 0,38 | 1,27 +/- 0,14 | 1,39 +/- 0,04 |
| HT29-Tumor | 8,34 +/- 1,85 | 5,86 +/- 1,33 | 5,01 +/- 0,87 | 5,70 +/- 0,50 |
| HPM73-Tumor | 4,89 +/- 2,62 | 5,49 +/- 1,45 | 4,75 +/- 1,18 | 6,74 +/- 1,97 |
| Knochen | 1,56 +/- 0,53 | 1,43 +/- 0,63 | 1,51 +/- 0,07 | 1,73 +/- 0,16 |
| Knochenmark | 2,51 +/- 1,03 | 2,77 +/- 1,08 | 2,90 +/- 0,27 | 3,15 +/- 0,60 |
| Restkörper | 2,73 +/- 0,64 | 3,11 +/- 0,63 | 2,76 +/- 0,22 | 2,96 +/- 0,20 |

EP 0 420 880 B1

## Tabelle 4 b

Tumor-Gewebe- = $\dfrac{\text{% d. Dosis/g HPM73-Tumor}}{\text{% d. Dosis/g Gewebe}}$
Quotient

| | Dosis I 5 µg/Tier | | | Dosis II 50 µg/Tier | | | Dosis III 250 µg/Tier | | | Dosis IV 1 mg/Tie | | |
|---|---|---|---|---|---|---|---|---|---|---|---|---|
| | MW | +/- | S | MW | +/- | S | MW | +/- | S | MW | +/- | S |
| Blut | 0,29 | +/- | 0,08 | 0,30 | +/- | 0,05 | 0,31 | +/- | 0,07 | 0,39 | +/- | 0,10 |
| Leber | 1,39 | +/- | 0,35 | 1,40 | +/- | 0,44 | 1,3 | +/- | 0,37 | 1,81 | +/- | 0,73 |
| Nieren | 1,53 | +/- | 0,36 | 1,43 | +/- | 0,12 | 1,39 | +/- | 0,35 | 1,85 | +/- | 0,42 |
| Milz | 1,80 | +/- | 0,32 | 1,99 | +/- | 0,37 | 1,89 | +/- | 0,36 | 2,15 | +/- | 0,61 |
| Muskel | 4.04 | +/- | 0,94 | 3,85 | +/- | 1,12 | 3,71 | +/- | 0,73 | 4,85 | +/- | 1,30 |
| Knochen | 3,06 | +/- | 0,69 | 4,17 | +/- | 1,38 | 3,14 | +/- | 0,74 | 3,94 | +/- | 1,27 |

Tumor-Gewebe- = $\dfrac{\text{% d. Dosis/g HT 29-Tumor}}{\text{% d. Dosis/g Gewebe}}$
Quotient

| | Dosis I 5 µg/Tier | | | Dosis II 50 µg/Tier | | | Dosis III 250 µg/Tier | | | Dosis IV 1 mg/Tier | | |
|---|---|---|---|---|---|---|---|---|---|---|---|---|
| | MW | +/- | S | MW | +/- | S | MW | +/- | S | MW | +/- | S |
| Blut | 0,52 | +/- | 0,05 | 0,32 | +/- | 0,04 | 0,33 | +/- | 0,07 | 0,33 | +/- | 0,03 |
| Leber | 2,51 | +/- | 0,55 | 1,49 | +/- | 0,45 | 1,47 | +/- | 0,33 | 1,50 | +/- | 0,23 |
| Nieren | 2,75 | +/- | 0,39 | 1,53 | +/- | 0,08 | 1,46 | +/- | 0,23 | 1,59 | +/- | 0,28 |
| Milz | 3,28 | +/- | 0,62 | 2,13 | +/- | 0,35 | 2,03 | +/- | 0,44 | 1,82 | +/- | 0,16 |
| Muskel | 7,36 | +/- | 1,65 | 4,09 | +/- | 1,04 | 3,99 | +/- | 0,99 | 4,12 | +/- | 0,44 |
| Knochen | 5,51 | +/- | 0,80 | 4,51 | +/- | 1,67 | 3,31 | +/- | 0,44 | 3,33 | +/- | 0,57 |

EP 0 420 880 B1

Beispiel 4:

Je 6 Nacktmäusen (Balb/c nu/nu 20 g, weiblich) wurden 5 bzw. 1000 µg des mit [125] J markierten mono-klonalen Antikörpers C 1 P 83 gelöst in jeweils 300 µl physiologischen Phosphatpuffers i.v. appliziert. Die Tiere trugen als Transplantate HT 29 Coloncarcinome und MKN 45 Magencarcinome. Die Versuche wurden 2 Wochen nach subcutaner Injektion vom jeweils $10^7$ Zellen durchgeführt.

48 Stunden nach Injektion wurden die Tiere getötet und folgende Organe entnommen: Blut, Leber, Nieren, Milz, Muskel, HT29 Tumor, MKN45 Tumor, Knochen, Knochenmark.

Wie die Tabelle 5 zeigt, nimmt die Konzentration (% der Dosis/g) des spezifischen Antikörpers in der hohen Dosierung gegenüber der niedrigen Dosierung sehr deutlich ab, während der nicht spezifische Tumor nur eine geringe Abnahme in der Konzentration zeigt.

Damit Konnte die zu erwartende Sättigung der Tumoranreicherung eines spezifischen Antikörpers gezeigt werden.

## Tabelle 5

### Konzentration des Antikörpers in den einzelnen Organen

#### ( % Dosis /g Organ )

| Gewebe | 5 µg | 1mg |
|---|---|---|
| Blut | 14,08 +/- 2,13 | 15,47 +/- 3,11 |
| Leber | 2,69 +/- 0,64 | 3,22 +/- 1,47 |
| Nieren | 3,46 +/- 0,68 | 3,36 +/- 0,79 |
| Milz | 2,40 +/- 0,55 | 2,60 +/- 1,13 |
| Muskel | 1,20 +/- 0,29 | 1,10 +/- 0,12 |
| Ht29-Tumor | 7,22 +/- 1,33 | 5,14 +/- 2,37 |
| MKN45-Tumor | 25,32 +/- 4,08 | 10,18 +/- 1,86 |
| Knochen | 1,46 +/- 0,27 | 1,50 +/- 0,39 |
| Knochenmark | +/- | 2,88 +/- 0,88 |
| Restkörper | 2,33 +/- 0,38 | 2,80 +/- 0,47 |

Kontrollversuch zu Beispiel 4:

Verteilung von [125]J-Avidin
in tumortragenden Nacktmäusen

Es wurden 3 Dosierungen (10 µg, 100 µg, 1 mg pro Tier, 3 Tiere pro Dosis) i.v. in Nacktmäusen (Balb/c nu/nu mit HT 29 Coloncarcinomen) appliziert. Das Protein lag in 300 µl 0,9 % Nacl gelöst vor. In Stoffwechselkäfigen wurden Harn und Faeces 4, 24 und 48 Std. nach Applikation gesammelt. Nach 48 Stunden wurden die Tiere getötet und folgende Organe entnommen: Blut, Leber, Nieren, Milz, Muskel, Tumor, Knochen und Knochenmark. Der Restkörper wurde in KOH gekocht. Die Messung der Aktivität erfolgt in einem Gamma-Counter. Tabelle 6 a zeigt die Avidin-Konzentration in den einzelnen Organen.

Die Gewebskonzentrationen steigen linear mit der Dosis an. Die Tumor-Gewebe-Quotienten (Tabelle 6b) liegen bis auf Muskel und Knochen unter 1, d.h. es findet keine Anreicherung im Tumor statt.

EP 0 420 880 B1

## Tabelle 6 a

### Avidin-Konzentrationen in den einzelnen Organen
( % Dosis / g Organ)

| Gewebe | Dosis I (10 µg/Tier) | Dosis II (100 µg/Tier) | Dosis III ( 1 mg/Tier |
|---|---|---|---|
| Blut | 0,27 +/- 0,04 | 0,21 +/- 0,04 | 0,23 +/- 0,06 |
| Leber | 0,42 +/- 0,09 | 0,27 +/- 0,06 | 0,23 +/- 0,04 |
| Nieren | 0,33 +/- 0,01 | 0,30 +/- 0,03 | 0,35 +/- 0,07 |
| Milz | 1,16 +/- 0,62 | 0,44 +/- 0,17 | 0,29 +/- 0,14 |
| Muskel | 0,03 +/- 0,00 | 0,02 +/- 0,00 | 0,02 +/- 0,01 |
| HT29-Tumor | 0,11 +/- 0,02 | 0,06 +/- 0,02 | 0,11 +/- 0,04 |
| Knochen | 0,15 +/- 0,02 | 0,09 +/- 0,01 | 0,09 +/- 0,02 |
| Knochenmark | 0,44 +/- 0,17 | 0,15 +/- 0,06 | 0,09 +/- 0,02 |
| Restkörper | 0,31 +/- 0,03 | 0,25 +/- 0,05 | 0,23 +/- 0,04 |

Tabelle 6b

$$\text{Tumor-Gewebe-Quotient} = \frac{\text{Dosis /g HT 29-Tumor}}{\text{Dosis /g Gewebe}}$$

| Gewebe | Dosis I (10 µg/Tier) | Dosis II ( 100 µg/Tier) | Dosis III (1 mg/Tier) |
|---|---|---|---|
| Blut | 0,40 +/- 0,03 | 0,37 +/- 0,02 | 0,45 +/- 0,06 |
| Leber | 0,27 +/- 0,07 | 0,29 +/- 0,07 | 0,48 +/- 0,12 |
| Nieren | 0,33 +/- 0,06 | 0,25 +/- 0,04 | 0,31 +/- 0,07 |
| Milz | 0,11 +/- 0,06 | 0,19 +/- 0,08 | 0,40 +/- 0,08 |
| Muskel | 4,30 +/- 0,37 | 3,62 +/- 0,29 | 5,11 +/- 0,78 |
| Knochen | 0,74 +/- 0,25 | 0,86 +/- 0,24 | 1,25 +/- 0,25 |

EP 0 420 880 B1

Beispiel 5:

Darstellung eines Coloncarcinoms auf einer Nacktmaus mittels eines Antikörperkomplexkonjugates

Einer Nacktmaus, Balb/c, nu/nu, mit HT 29-Coloncarcinom wurde ein Antikörperkomplexkonjugat mit 50 Gd-Atomen pro Antikörper gelöst in 0,9% Nacl i.v. appliziert.

Zuvor war das Tier in einem 2 Tesla Kernspintomographen mit einer Spin-Echo-Sequenz ($T_R$= 400 msec, $T_E$ = 30 msec) im Bereich des Tumors im Transversalschnitt gemessen worden (Bild 1).

24 Stunden nach der Applikation wurde in derselben Ebene eine zweite Aufnahme mit Spin-Echo-Sequenz ($T_R$ = 400 msec, $T_E$ = 30 msec) gemacht ( Bild 2). Im Bereich des Tumors ist eine deutliche Signalzunahme feststellbar.

Der verwendete Antikörper hatte keine Tumorspezifität (DP 43-16 Product Code MCA 220 Camon Laborservice GmbH, Wiesbaden; Antigen: Neurofilament 160 K vom Menschen).

Hinterlegungsdaten:

mAK C1 - P83     ATCC 40575 (08.02.89)
mAK 17 - 1A     ATCC 40576 (08.02.89)
Zellinie MKN 45     88072002 (20.07.88)
bei PHLS
Center for applied microbiology
and research, division of biologics
Porton Down
Salisbury
Wiltshire, SP4 0JG
United Kingdom

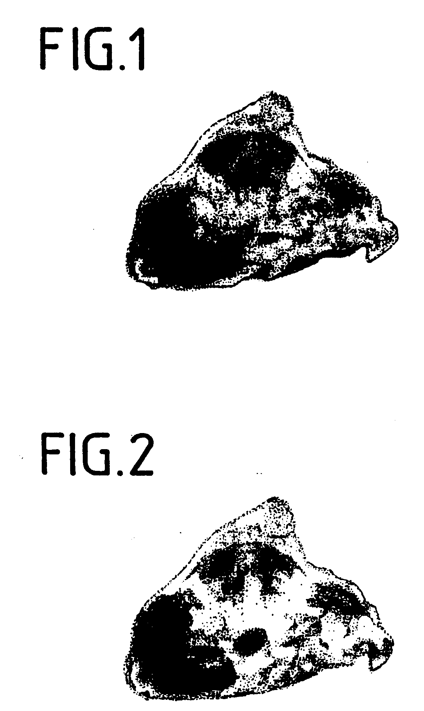

## Patentansprüche

1. Verfahren zur bildlichen Darstellung von Tumoren unter Verwendung von Konjugaten monoklonaler Antikörper oder deren Fragmenten mit Metallkomplexen oder mit jodierten Verbindungen, **dadurch gekennzeichnet,** daß die verwendeten monoklonalen Antikörper oder deren Fragmente keine definierte Bindungsspezifität hinsichtlich der tumorassoziierten Antigene aufweisen und die in den Komplexen vorhandenen Metalle Chrom (III), Mangan (II), Eisen (II), Cobalt (II), Nickel (II), Kupfer (II), Praseodym (III), Neodym (III), Samarium (III), Ytterbium (III), Gadolinium (III), Terbium (III), Dysprosium (III), Holmium (III), Erbium (III) oder Eisen (III) sind.

2. Verfahren zur bildlichen Darstellung von Tumoren nach Anspruch 1, **dadurch gekennzeichnet,** daß monoklonale Antikörper murinen Ursprungs verwendet werden.

3. Verfahren zur bildlichen Darstellung von Tumoren nach Anspruch 1, **dadurch gekennzeichnet,** daß monoklonale Antikörper humanen Ursprungs verwendet werden.

4. Verfahren zur bildlichen Darstellung von Tumoren nach anspruch 1 bis 3, **dadurch gekennzeichnet,** daß die monoklonalen Antikörper oder deren Fragmente über -NH$_2$-, -SH-, -COOH- oder -CHO-Gruppen an Avidin gekoppelt und in Form der entsprechenden Konjugate verwendet werden.

5. Verfahren zur bildlichen Darstellung von Tumoren nach Anspruch 1 bis 4, **dadurch gekennzeichnet,** daß die den Tumor darstellenden Diagnostika in zwei Schritten appliziert werden, wobei im ersten Schritt monoklonale Antikörper mit einer Spezifität gegenüber dem eigentlichen Diagnostikum verabfolgt werden, und im zweiten Schritt das Diagnostikum selbst verabfolgt wird.

6. Verfahren zur bildlichen Darstellung von Tumoren nach Anspruch 5, **dadurch gekennzeichnet,** daß im ersten Schritt monoklonale Antikörper mit einer Spezifität gegenüber Metallkomplexen intravenös appliziert werden und nach Absinken des Blutspiegels entsprechende Metallkomplexe verabfolgt werden.

7. Verfahren zur bildlichen Darstellung von Tumoren nach Anspruch 5, **dadurch gekennzeichnet,** daß im ersten Schritt mit Avidin bzw. Biotin konjugierte monoklonale Antikörper appliziert und im zweiten Schritt mit Biotin bzw. Avidin gekoppelte Metallkomplexe verabfolgt werden.

EP 0 420 880 B1

**8.** Verwendung der Verfahren nach Anspruch 1 bis 7 in der Kernspintomographie.

**9.** Verwendung der Verfahren nach Anspruch 1 bis 7 in der Röntgendiagnostik.

**Claims**

**1.** Method for the visual display of tumors using conjugates of monoclonal antibodies or their fragments with metal complexes or with iodinated compounds, characterised in that the used monoclonal antibodies or their fragments exhibit no defined binding specificity for tumor-associated antigens and the metals present in the complexes are chromium (III), manganese (II), iron (II), cobalt (II), nickel (II), copper (II), praseodymium (III), neodymium (III), samarium (III), ytterbium (III), gadolinium (III), terbium (III), dysprosium (III), holmium (III), erbium (III) or iron (III).

**2.** Method for the visual display of tumors according to claim 1, characterised in that monoclonal antibodies of murine origin are used.

**3.** Method for the visual display of tumors according to claim 1, characterised in that monoclonal antibodies of human origin are used.

**4.** Method for the visual display of tumors according to claim 1 to 3, characterised in that the monoclonal antibodies or their fragments are coupled by -NH$_2$ -, -SH -, -COOH - or -CHO - groups on to avidin and are used in form of corresponding conjugates.

**5.** Method for the visual display of tumors according to claim 1 to 4, characterised in that the diagnostic agents visualising the tumor are administered in two steps, whereby in the first step the monoclonal antibodies, with a specificity for the actual diagnostic agent, are administered, and in the second step the diagnostic agent itself is administered.

**6.** Method for the visual display of tumors according to claim 5, characterised in that during the first step monoclonal antibodies, with a specificity for metal complexes, are intravenously administered, and corresponding metal complexes are administered after decrease of the blood level.

**7.** Method for the visual display of tumors according to claim 5, characterised in that during the first step monoclonal antibodies conjugated with avidin or biotin are administered and during the second step metal complexes coupled on to biotin or avidin are administered.

**8.** Use of the methods according to claim 1 to 7 in nuclear spin tomography.

**9.** Use of the methods according to claim 1 to 7 in X-ray diagnosis.

**Revendications**

**1.** Procédé de mise en images, dite imagerie médicale, de tumeurs, avec des conjugués d'anticorps monoclonaux ou de leurs fragments et de complexes de métaux ou de composés iodés, procédé caractérisé en ce que les anticorps monoclonaux employés ou leur fragments ne présentent aucune spécificité de liaison définie pour les antigènes qui sont associés à la tumeur, et en ce que les métaux des complexes sont le chrome (III), le manganèse (II), le fer (II), le cobalt (II), le nickel (II), le cuivre (II), le praséodyme (III), le néodyme (III), le samarium (III), l'ytterbium (III), le gadolinium (III), le terbium (III), le dysprosium (III), l'holmium (III), l'erbium (III) ou le fer (III).

**2.** Procédé selon la revendication 1, caractérisé en ce que les anticorps monoclonaux utilisés sont d'origine murine.

**3.** Procédé selon la revendication 1, caractérisé en ce que les anticorps monoclonaux utilisés sont d'origine humaine.

**4.** Procédé selon les revendications 1 à 3, caractérisé en ce que les anticorps monoclonaux ou leurs fragments sont couplés à de l'avidine par des groupe -NH$_2$-, -SH-, -COOH- ou -CHO- et ils sont utilisés en

13

les conjugués correspondants.

5. Procédé selon les revendications 1 à 4, caractérisé en ce que les diagnostics représentant la tumeur sont appliqués en deux temps, à savoir que dans un premier temps sont donnés des anticorps monoclonaux ayant une spécificité pour le diagnostic spécifique proprement dit, et dans le second temps est donné le diagnostic lui-même.

6. Procédé selon la revendication 5, caractérisé en ce que dans le premier temps sont donnés par voie intraveineuse des anticorps monoclonaux ayant une spécifité pour des complexes de métaux, et après abaissement du taux sanguin sont donnés des complexes de métaux correspondants.

7. Procédé selon la revendication 5, caractérisé en ce que dans le premier temps sont donnés des anticorps monoclonaux conjugués avec de l'avidine et/ou de la biotine, et dans le second temps sont donnés des complexes de métaux couplés avec de la biotine et/ou de l'avidine.

8. Application des procédés selon les revendications 1 à 7 pour des tomographies par résonance magnétique nucléaire.

9. Application des procédés selon les revendications 1 à 7 pour des diagnostics radiographiques, c'est-à-dire par rayons X.

# FIG.1

# FIG.2